# FASCICULE DE BREVET EUROPEEN

(11) **EP 3 003 250 B1**
(45) Date de publication et mention de la délivrance du brevet: **18.12.2019**
(21) Numéro de dépôt: 14734876.7
(22) Date de dépôt: 06.06.2014
(51) Int. Cl.: A61K 8/24, A61K 8/49, A61Q 5/12, A61Q 19/00

(54) **PROCEDE DE TRAITEMENT DES FIBRES KERATINIQUES PAR FORMATION D'UN LIQUIDE IONIQUE**
VERFAHREN ZUR BEHANDLUNG VON KERATINHALTIGEN FASERN DURCH BILDUNG EINER IONISCHEN FLÜSSIGKEIT
METHOD OF TREATING KERATIN FIBRES BY FORMING AN IONIC LIQUID

(30) Priorité: 07.06.2013 FR 1355252
(43) Date de publication de la demande: 13.04.2016
(73) Titulaire: L'OREAL, 75008 Paris (FR)
(72) Inventeur: SAMAIN, Henri, F-91570 Bievres (FR)
(74) Mandataire: L'Oreal
(86) Numéro de dépôt international: PCT/FR2014/051373
(87) Numéro de publication internationale: WO 2014/195659

(56) Documents cités:
- EP-A1- 2 062 565
- WO-A1-2010/125302
- DE-A1- 10 008 640
- FR-A1- 2 978 038

## Description

La présente invention concerne un procédé de traitement cosmétique des matières kératiniques, notamment les cheveux, pour apporter un effet rémanent aux matières kératiniques.

Dans le domaine cosmétique, il est très souvent recherché d'améliorer la pénétration des actifs cosmétiques dans les cheveux afin d'obtenir des résultats cosmétiques améliorés. Une des voies qui permet d'améliorer la pénétration d'actifs est de les associer à des composés gras, souvent en quantité importante. Cette approche qui peut être efficace présente cependant l'inconvénient de laisser sur les cheveux un toucher gras qui est souvent inesthétique.

Une autre façon d'améliorer la pénétration d'actifs est l'utilisation de tensio-actifs. Les corps gras sont alors compatibles avec un milieu aqueux. Selon cette approche, on obtient souvent des touchers collants, peu cosmétiques.

Par ailleurs, ces deux approches ne sont pas totalement satisfaisantes en terme de niveau de pénétration dans les cheveux.

Il est donc important de parvenir à développer des systèmes qui permettent une meilleure pénétration d'actifs dans les matières kératiniques tout en conservant un toucher cosmétique agréable pour l'utilisateur, en particulier un toucher non gras, non luisant et/ou non collant.

On peut ainsi en fonction des actifs choisis envisager d'améliorer l'aspect des cheveux par exemple pour réparer ou modifier l'état des matières kératiniques lorsqu'elles sont endommagées, pour renforcer les cheveux, pour améliorer la rémanence de la coloration, pour donner du volume aux cheveux, pour lisser ou friser les cheveux , etc.

L'objet de la présente invention est de proposer un procédé de traitement des matières kératiniques qui permet d'améliorer la pénétration d'actifs cosmétiques tout en conservant un toucher cosmétique.

Ainsi, l'invention a pour objet un procédé de traitement des fibres kératiniques qui comprend i) l'application d'une composition comprenant au moins un premier liquide ionique hydrosoluble A⁺X⁻ comprenant un cation organique A⁺ et ii) l'application d'une composition comprenant un sel hydrosoluble B⁻Y⁺ comprenant un anion B⁻, l'anion B⁻ étant tel que par échange d'ions avec le premier liquide ionique A⁺X⁻, il se forme *in situ* sur les fibres un second liquide ionique A⁺B⁻ hydrophobe.

On applique le procédé de l'invention sur des fibres kératiniques par exemple les cheveux.

Dans le procédé de l'invention, les étapes i) et ii) peuvent être mise en œuvre indifféremment de l'ordre.

Par liquide ionique, on entend au sens de la présente invention un sel d'une molécule organique, ledit sel ayant une température de fusion inférieur ou égale à 150°C, de préférence inférieure à 100°C. De préférence, le sel reste liquide jusqu'à 300°C, et plus préférentiellement le sel est liquide à la température ambiante, c'est-à-dire à une température de fusion inférieure ou égale à 50°C et supérieure à 0°C.

La température de fusion est mesurée par analyse calorimétrique différentielle, avec une vitesse de montée en température de 10°C/minute, la température de fusion est alors à une température correspondant au sommet du pic endotherme de fusion obtenu lors de la mesure.

Par liquide ionique hydrophobe, on entend selon l'invention un liquide ioniques présentant une solubilité dans l'eau à température ambiante (25°C) inférieure à 5 %, de préférence inférieure à 1 % en poids, voire inférieure à 0,5 %. Dans le cadre de l'invention, le caractère hydrophobe du liquide ionique est tel qu'il démixe dans l'eau.

Dans le cadre de l'invention, on entend par hydrosoluble, un sel ou un liquide ionique qui présente une solubilité dans l'eau à 25°C supérieure à 5 % de préférence encore supérieure à 10 %, c'est-à-dire formant à cette concentration un milieu macroscopiquement homogène, transparent et isotrope

Les liquides ioniques utilisés selon l'invention possèdent un cation A+ minéral ou organique, de préférence organique choisi parmi les cations imidazolium, pyrazolium, pyridinium, pyrimidinium, tétra-alkyl(C₁-C₆)phosphonium, tétra-alkyl(C₁-C₆)ammonium, guanidinium, cholinium, pyrrolidinium, uronium, thiouronium et isothiouronium.

A titre d'exemple, les cations imidazolium peuvent répondre à la formule suivante : 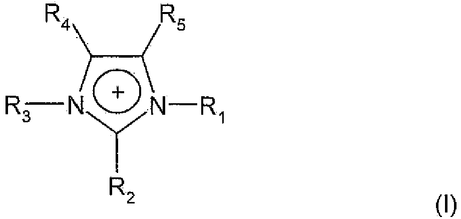
dans laquelle R₁ représente un groupe alkyle comportant de 1 à 15 atomes de carbone, de préférence de 1 à 6 atomes de carbone, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R₂, R₃, R₄, R' et R₅, identiques ou différents, représentent chacun un atome d'hydrogène ou un groupe alkyle comportant de 1 à 5 atomes de carbone, de préférence de 1 à 4 atomes de carbone , ou un groupe aryle en C₆-C₃₀, éventuellement substitué par un ou plusieurs groupes alkyles en C₁-C₄.

De préférence, R₁ et R₃, identiques ou différents, représentent chacun un groupe alkyle comportant de 1 à 4 atomes de carbone.

Les cations peuvent répondre à l'une des formules suivantes : 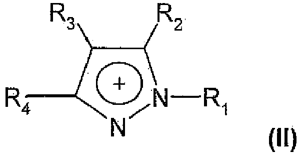 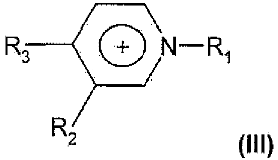 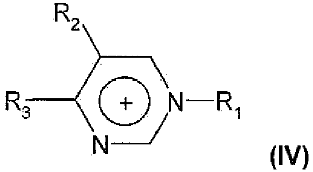 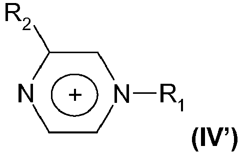 dans lesquelles R₁ R₂, R₃ et R', identiques ou différents, sont tels que définis précédemment.

Les cations tétra-alkyl(C₁-C₆)phosphonium peuvent répondre à la formule suivante : 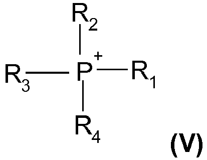
dans laquelle R₁, R₂ et R₃ et R₄, identiques ou différents, représentent un groupe alkyle comportant de 1 à 15 atomes de carbone et de préférence de 1 à 6 atomes de carbone, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄.

Les cations tétra-alkyl(C₁-C₆)ammonium répondent en particulier à la formule suivante : 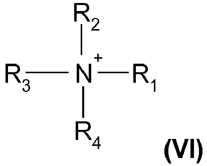
dans laquelle R₁, R₂, R₃, R₄, identiques ou différents, représentent un groupe alkyle comportant de 1 à 6 atomes de carbone, de préférence de 1 à 4 atomes de carbone, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄. De préférence le groupement alkyle de R₁, R₂, R₃, R₄ n'est pas substitué.

Les cations guanidinium peuvent répondre à la formule suivante : 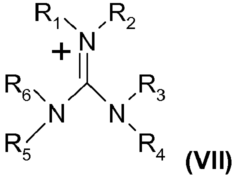
dans laquelle R₁ à R₆, identiques ou différents, représentent un atome d'hydrogène ou un groupe alkyle comportant de 1 à 15 atomes de carbone, de préférence de 1 à 6 atomes de carbone, éventuellement substitué par un ou plusieurs groupements aryles aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄.

Les cations cholinium peuvent répondre à la formule suivante : 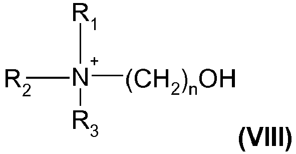
dans laquelle R₁, R₂ et R₃, identiques ou différents, représentent chacun un groupe alkyle comportant de 1 à 15 atomes de carbone, de préférence de 1 à 6 atomes de carbone, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄,
n est un entier variant de 1 à 15, de préférence de 1 à 12.

Les cations pyrrolidinium peuvent répondre à la formule : 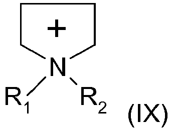
dans laquelle R₁ représente un groupe alkyle comportant de 1 à 15 atomes de carbone, de préférence de 1 à 6 atomes de carbone, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R₂ et R' représentent indépendamment l'un de l'autre un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄.

Les cations isouronium peuvent répondre à la formule suivante : 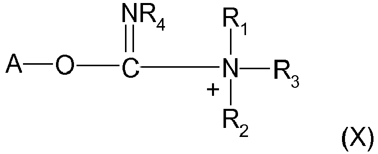
dans laquelle R₁ à R₄ et A, identiques ou différents, représentent chacun un groupe alkyle en C₁-C₁₅, de préférence en C₁-C₆, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄.

Les cations isothiouronium peuvent répondre à la formule suivante : 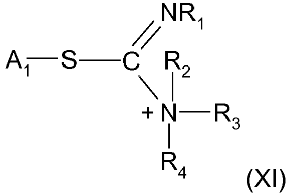
dans laquelle R₁ à R₄ et A₁, identiques ou différents, représentent chacun un groupe alkyle en C₁-C₁₅, de préférence en C₁-C₆, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄.

Les cations uronium peuvent répondre à la formule suivante : 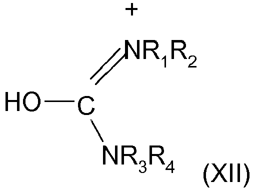
dans laquelle R₁ à R₄, identiques ou différents, représentent chacun un groupe alkyle en C₁-C₁₅, de préférence en C₁-C₆, éventuellement substitué par un ou plusieurs groupements aryles en C₆-C₃₀, thiols, hydroxy ou interrompu par un ou plusieurs atomes d'oxygène ou de soufre ou par un ou plusieurs groupements NR',
R' représente un atome d'hydrogène ou un groupe alkyle comportant de 1 à 5 atomes de carbone et de préférence de 1 à 4 atomes de carbone, ou un groupe aryle en C₆-C₃₀, éventuellement substitué par un ou plusieurs groupes alkyles en C₁-C₄.

Les cations A⁺ peuvent être constitués d'actifs cosmétiques comprenant un radical ammonium quaternaire. A titre d'exemple, on peut citer les filtres solaires de type merocyanine, les colorants azoïque, les colorants cationiques.

De préférence, le cation A⁺ des liquides ioniques utilisés selon l'invention est choisi parmi les cations ammonium, imidazolium et cholinium. Selon un mode de réalisation, ces cations peuvent être substitués par des actifs cosmétiques tels décrits ci-dessus.

Parmi les cations utilisables, on peut citer 1-méthyl-imidazolium ; 1-butylimidazolium ; 3-méthyl-1-tétradécyl-imidazolium ; 1-hexadécyl-3-méthyl-imidazolium ; 1-phénylpropyl-3-méthyl-imidazolium ; 1-éthyl-2,3-diméthylimidazolium ; 1-propyl-2,3-diméthyl-imidazolium ; 1-butyl-2,3-diméthylimidazolium ; 1-hexadécyl-2,3-diméthyl-imidazolium ; N-éthylpyridinium ; N-butyl-3-méthyl-pyridinium ; 4-méthyl-N-butyl-pyridinium ; N-butyl-3,4-diméthyl-pyridinium ; 3-éthyl-N-butyl-pyridinium ; N-hexyl-pyridinium ; 3-méthyl-N-hexylpyridinium ; 4-méthyl-N-hexylpyridinium ; N-octyl-pyridinum ; 3-méthyl-N-octyl-pyridinium ; 4-méthyl-N-octyl-pyridinium ; tétrabutyl-phosphonium ; Tri-isobutyl(méthyl)-phosphonium ; benzyltriphényl-phosphonium ; éthyl(tributyl)phosphonium ; méthyl(tributyl)phosphonium ; tétraoctylphosphonium ; tétraméthylammonium ; 1,1-diméthyl-pyrrolidinium ; 1-éthyl-1-méthylpyrrolidinium ; 1,1-dipropylpyrrolodinium ; 1-butyl-1-méthyl-pyrrolidinium ; 1-butyl-1-éthyl-pyrrolidinium ; 1,1-dibutyl-pyrrolidinium ; 1-hexyl-1-méthyl-pyrrolidinium ; 1,1-dihexyl-pyrrolidinium ; 1-octyl-1-méthyl-pyrrolidinium ; guanidinium ; N,N,N',N'-tétraméthyl-N"-éthylguanidinium ; N-pentaméthyl-N-isopropyl-guanidinium ; hexaméthylguanidinium ; O-méthyl-N,N,N',N'-tétraméthylisouronium ; S-éthyl-N,N,N',N'-tétraméthylisothiouronium ; 1-butyl-3-éthyl-imidazolium.

Selon la présente invention, le cation Y+ peut être choisi parmi les monocations, tels que ceux des métaux alcalins, les amines et ammoniums ou autres dérivés azotés tels que les guanidiniums
Les liquides ioniques A⁺X⁻ ou A⁺B⁻ possèdent un anion B⁻ ou X⁻ minéral ou organique.

B⁻ est notamment choisi parmi les anions peu coordonnant tels que les anions alkylpolyhalogénés ou polyarylés, les anions à chaine hydrophobe carbonée, les alkysulfates à chaines longues, de préférence supérieur à 5 atomes de carbone, les ions halogénophosphates, les ions (di/tri)halogénoacétates les ions (di/tri)halogénomethane sulfonates, les ions halogénosulfates, l'halogène étant de préférence le fluor. A titre d'exemple d'anions B⁻, on peut citer l'hexafluorophosphate, le tetrafluoroborate, les tétraarylborates, le bistriflimidure, les alkysulfates à chaines > C5, tel que l'octylsulfate(C₈H₁₇OSO₃₋), l'ion tris(pentafluoroéthyl)trifluorophosphate ; l'ion triflate [TfO] (CF₃SO₂⁻), l'ion nonaflate [NfO] (CF₃(CF₂)₃SO₂⁻), un ion bis(trifyl)amide [Tf₂N] ((CF₃SO₂)₂N⁻) ; les ions trifluoroacétate [TA] (CF₃CO₂⁻), et heptafluorobutanoate [HB] (CF₃(CF₂)₃CO₂⁻) ; l'ion trifluorométhanesulfonate (CF₃SO₃⁻) ; les ions dicyanamide, salicylate.

L'anion X- est en général choisi parmi les chlorures et les bromures, les carboxylates à chaine courte, notamment C1-C4, les nitrates, les composés oxydes anioniques. A titre d'exemple, on peut citer les anions chlorure (Cl⁻), bromure (Br⁻) ; les ions tétrahalogénoaluminates tels que le tétrachloroaluminate (AlCl₄⁻) ; les ions tétrahalogénonickel tels que le tétrachloronickel (NiCl₄⁻), l'ion perchlorate (ClO₄⁻), l'ion nitrate (NO₃⁻) ; l'ion nitrite (NO₂⁻) ; l'ion sulfate (SO₄²⁻), l'ion hydrogénosulfate, l'ion phosphate, et les ions dérivés de l'ion phosphate, les acétates et les formiates. Il peut être aussi choisi parmi les anions organiques tels que l'ion méthylsulfate (CH₃SO₄⁻), dibutylphosphate, les ions citrate, lactate.

De manière non exhaustive, les liquides ioniques A⁺X⁻ utilisés selon l'invention peuvent être choisis parmi les composés suivants :
le chlorure de 1-éthyl-3-méthylimidazolium,
le bromure de 1-éthyl-3-méthylimidazolium,
le chlorure de 1-butyl-3-méthylimidazolium,
le chlorure de 1-hexyl-3-méthylimidazolium,
le chlorure de 1-méthyl-3-octylimidazolium,
le chlorure de 1-décyl-3-méthylimidazolium,
le bromure de 1-décyl-3-méthylimidazolium,
le chlorure de 1-dodécyl-3-méthylimidazolium,
le chlorure de 1-méthyl-3-tétradécylimidazolium,
le chlorure de 4-méthyl-N-butyl-pyridinium,
le chlorure de 3-méthyl-N-butylpyridinium,
le chlorure de 4-méthyl-N-hexylpyridinium,
le méthylsulfate de 1,3-diméthylimidazolium,
le méthylsulfate de 1-méthyl-3-butylimidazolium,
l'acétate de 1-éthyl-3-méthylimidazolium,
le sulfate de 1-éthyl-3-méthylimidazolium,
le bromure de 1-butylpyridinium,
le trifluorométhanesulfonate de 1-butylpyrimidinium,
le trifluorométhanesulfonate de 1-hexylpyrimidinium,
le trifluoroacétate de 1-éthyl-3-méthylimidazolium,
le chlorure de trihexyl-tétradécyl-phosphonium,
le chlorure de tributyl-tétradécyl-phosphonium,
le trifluoroacétate de 1-éthyl-3-méthylimidazolium.
le chlorure de 1-hexyl-2,3-diméthylimidazolium,
le chlorure de 1-éthyl-2,3-diméthylimidazolium,
le dicyanamide de 1-éthyl-3-méthylimidazolium,
l'hydroxyde de tétrabutylammonium,
le salicylate de choline,
le méthylsulfate de tributylméthylammonium,
l'acétate de cholinium,
l'acétate de tétraéthylammonium tétrahydraté,
le dibutylphosphate de triéthylméthylammonium,
le L-(+)lactate de 1-éthyl-3-méthylimidazolium,
et leurs hydrates.

De manière non exhaustive, les sels B⁻Y⁺ utilisés selon l'invention peuvent être choisis parmi les composés suivants :
Hexafluorophosphate d'ammonium, ou de métaux alcalins (Na+....)
Tétrafluoroborate d'ammonium, ou de métaux alcalins
Bistriflimidure d'ammonium ou de métaux alcalins
Octylsulfate d'ammonium ou de métaux alcalins

Selon un mode de réalisation particulier le liquide ionique A⁺B⁻ formé au moment de l'application est liquide ou pâteux à température ambiante.

A titre d'exemple de liquide ionique hydrophobe A⁺B⁻, on peut citer l'hexafluorophosphate de 3-méthyl octylimidazolium, l'hexafluorophosphate de arylazodimethylamidazolium, l'hexafluorophosphate de 3-méthyl butylimidazolinium, l'hexafluorophosphate de 1-éthyl-3-méthylimidazolium, l'hexafluorophosphate de 1-hexyl-3-méthylimidazolium, l'hexafluorophosphate de 1-décyl-3-méthylimidazolium, l'hexafluorophosphate de 1-dodécyl-3-méthylimidazolium, l'hexafluorophosphate de 1,3-diméthylimidazolium, l'hexafluorophosphate de de 4-méthyl-N-butyl-pyridinium, l'hexafluorophosphate de 3-méthyl-N-butylpyridinium, l'hexafluorophosphate de 4-méthyl-N-hexylpyridinium, l'hexafluorophosphate de 1-éthyl-3-méthylimidazolium, l'hexafluorophosphate de 1-butylpyridinium, de l'hexafluorophosphate d' arylazodimethylimidazolium, de l'hexafluorophosphate d'arylazométhylpyridinium

Les liquides ioniques représentent généralement de 1 à 100%, de préférence de 10 à 100%, mieux de 40 à 100%, en poids par rapport au poids total de la composition de l'invention.

La composition cosmétique utilisée selon l'invention peut comprendre en outre un ou plusieurs composés liquides à température ambiante autre que les liquides ioniques utiles dans le procédé de l'invention.

Le composé liquide est de préférence un solvant et en particulier un solvant choisi parmi l'eau, les alcools aliphatiques en C1-C4, tel que l'éthanol et l'isopropanol, les solvants organiques solubles ou dispersibles dans l'eau, tel que l'acétone, le propylène carbonate, l'alcool benzylique, les dérivés d'éther de glycol, le propylène glycol, les polyols tels que le glycérol, et les polyéthylènes glycol.

Préférentiellement, le composé liquide est un solvant polaire.

On a constaté que le procédé selon l'invention permet d'obtenir une plus grande pénétration dans les matières kératiniques des liquides ioniques comparativement aux procédés de l'état de la technique qui comprennent l'application directe du liquide ionique lipophile. Ainsi avec le procédé de l'invention, on obtient un procédé de traitement des matières kératiniques avec des liquides ioniques présentant une meilleure efficacité. On peut ainsi envisager en fonction du liquide ionique formé in situ une efficacité supérieure par exemple lors de l'application sur les fibres kératiniques pour le renforcement et/ou la réparation des cheveux, la coloration des cheveux, le conditionnement des cheveux en particulier pour apporter ou améliorer le démêlage, le lissage, la peignabilité, la maniabilité, la douceur de la chevelure et de façon plus durable.

Le procédé peut également comprendre au moins une étape de rinçage.

Lorsque le procédé de l'invention est appliqué aux fibres kératiniques, en particulier les cheveux, il peut comprendre une étape de traitement thermique. Ainsi, l'application de la composition sur les cheveux peut être éventuellement suivie d'une étape de rinçage et/ou d'une étape de traitement thermique, par exemple à une température comprise entre 150°C et 250°C.

Le procédé de l'invention peut comprendre une étape de rinçage intermédiaire entre l'application du ou des liquides ioniques A⁺X⁻ et du sel B⁻Y⁺. Il peut aussi comprendre un rinçage final. Selon un mode de réalisation particulier, le procédé de l'invention ne comprend pas de rinçage final.

Les compositions utiles dans le procédé de l'invention peuvent en outre comprendre au moins un ingrédient cosmétique usuel, notamment choisi parmi les propulseurs; les huiles ; les corps gras solides et notamment les esters en C₈-C₄₀ ; les acides en C₈-C₄₀ ; les alcools en C₈-C₄₀ ; les tensioactifs non ioniques ; les tensioactifs cationiques ; les tensioactifs anioniques ; les tensioactifs amphotères ; les tensioactifs zwittérioniques ; les filtres solaires ; les agents hydratants ; les agents antipelliculaires ; les agents antioxydants ; les agents chélatants ; les agents nacrants et opacifiants ; les agents plastifiants ou de coalescence ; les hydroxyacides ; les charges ; les silicones et en particulier les polydiméthylsiloxanes ; les épaississants, polymériques ou non ; les gélifiants ; les émulsionnants ; les polymères notamment conditionneurs ou coiffants ; les parfums ; les agents d'alcalinisation ou d'acidification ; les silanes ; les agents de réticulation. La composition peut bien évidemment comprendre plusieurs ingrédients cosmétiques figurant dans la liste ci-dessus.

Selon leur nature et la destination des compositions, les ingrédients cosmétiques usuels peuvent être présents en des quantités usuelles, aisément déterminables par l'homme du métier, et qui peuvent être comprises, pour chaque ingrédient, entre 0,01 à 80% en poids.

### Exemple

### Exemple 1 :

On réalise deux compositions suivantes :
Composition **a** :
   - Chlorure de 3-méthyl-octylimidazolium (CAS 64697-40-1) à 13,5%
   - eau 90%
Composition **b** :
   - hexafluorophosphate d'ammonium (CAS 16941-11-0) à 10%
   - Eau 90%

Les deux compositions sont parfaitement claires et solubles.

Quand on mélange les deux compositions, on obtient un liquide ionique à base d'hexafluorophosphate de 3-méthyl-octylimidazolium, qui n'est pas soluble dans l'eau. Ainsi, immédiatement après mélange, on obtient un mélange à 2 phases.

Conformément à notre invention, on applique d'abord la composition **a** sur les cheveux, puis, 5mn plus tard, on applique la composition **b**.

A titre de comparaison, on applique une composition **c** contenant 10% d' hexafluorophosphate 3-méthyl-octylimidazolium (liquide ionique) et 90% d'eau.

L'application successive de la composition **a** puis **b**, donne un résultat beaucoup moins gras que l'application de la composition **c** sur les cheveux.

Après rinçage et lavage des cheveux, on obtient un cheveu beaucoup doux, qu'avec le procédé de l'invention comparé à l'application de la composition **c**.

### Exemple 2 :

On réalise la composition suivante :
Composition **d** :
- Chlorure de 3-méthyl-butylimidazolium (CAS 64697-40-1) à 10%
- eau 90%

On réalise aussi la composition **b** de l'exemple 1.

Les deux compositions sont parfaitement claires et solubles.

Quand on mélange les deux compositions, on obtient un liquide ionique à base d'hexafluorophosphate de 3-méthyl-butylimidazolium, qui n'est pas soluble dans l'eau. Ainsi, immédiatement après mélange, on obtient un mélangé à 2 phases.

Conformément à notre invention, on applique d'abord la composition **d** sur les cheveux, puis, 5mn plus tard, on applique la composition **b**.

A titre de comparaison, on applique une composition **e** contenant 10% d'hexafluorophosphate 3-méthyl-butylimidazolium (liquide ionique) et 90% d'eau.

L'application successive de la composition **a** puis **b**, donne un résultat beaucoup moins gras que l'application de la composition **e** sur les cheveux.

Après rinçage et lavage des cheveux, on obtient un cheveu beaucoup doux, qu'avec le procédé de l'invention comparé à l'application de la composition **c**.

## Revendications

1. Procédé de traitement des fibres kératiniques qui comprend i) l'application d'une composition comprenant au moins un premier liquide ionique_hydrosoluble A⁺X⁻ présentant une solubilité dans l'eau à 25°C supérieure à 5% en poids comprenant un cation organique A⁺ et ii) l'application d'une composition comprenant un sel hydrosoluble B⁻Y⁺ présentant une solubilité dans l'eau à 25°C supérieure 5 % en poids comprenant un anion B⁻, l'anion B⁻ étant tel que par échange d'ions avec le premier liquide ionique A⁺X⁻, il se forme *in situ* sur les fibres un second liquide ionique A⁺B⁻ hydrophobe présentant une solubilité dans l'eau à température ambiante (25°C) inférieure à 5 % en poids ; les liquides ioniques ayant une température de fusion inférieure ou égale à 150°C.

2. Procédé selon la revendication 1 dans lequel le premier et le second liquide ionique présente une température de fusion inférieure ou égale à 50°C et supérieure à 0°C.

3. Procédé selon la revendication 1 ou 2 dans lequel le cation A⁺ est un cation organique choisi parmi les cations imidazolium, pyrazolium, pyridinium, pyrimidinium, tétra-alkyl(C₁-C₆)phosphonium, tétra-alkyl(C₁-C₆)ammonium, guanidinium, cholinium, pyrrolidinium, uronium, thiouronium et isothiouronium

4. Procédé selon la revendication 1 ou 2 dans lequel le cation Y⁺ est choisi parmi les monocations, tels que ceux des métaux alcalins, les amines et ammoniums ou autres dérivés azotés tels que les guanidiniums.

5. Procédé selon la revendication 1 ou 2 dans lequel les anions B⁻ sont choisis parmi les anions peu coordonnant choisis parmi les anions alkylpolyhalogénés ou polyarylés, les anions à chaine hydrophobe carbonée, les alkysulfates à chaines longues supérieure à 5 atomes de carbone, les ions halogénophosphates, les ions (di/tri)halogénoacétates les ions (di/tri)halogénométhane sulfonates, les ions halogénosulfates.

6. Procédé selon la revendication 1 ou 2 dans lequel les anions X⁻ sont choisis parmi les bromures, les chlorures, les carboxylates à chaine courte en C1-C4, les nitrates, les composés oxydes anioniques, les ions tétrahalogénoaluminates, les ions tétrahalogénonickel, l'ion perchlorate (ClO₄⁻), l'ion nitrate (NO₃⁻) ; l'ion nitrite (NO₂⁻) ; l'ion sulfate (SO₄²⁻), l'ion hydrogénosulfate, l'ion phosphate, les acétates et les formiates, les ions citrate, lactate.

7. Procédé selon l'une quelconque des revendications précédentes dans lequel A⁺X⁻ est choisi parmi les composés suivants :
le chlorure de 1-éthyl-3-méthylimidazolium,
le bromure de 1-éthyl-3-méthylimidazolium,
le chlorure de 1-butyl-3-méthylimidazolium,
le chlorure de 1-hexyl-3-méthylimidazolium,
le chlorure de 1-méthyl-3-octylimidazolium,
le chlorure de 1-décyl-3-méthylimidazolium,
le bromure de 1-décyl-3-méthylimidazolium,
le chlorure de 1-dodécyl-3-méthylimidazolium,
le chlorure de 1-méthyl-3-tétradécylimidazolium,
le chlorure de 4-méthyl-N-butyl-pyridinium,
le chlorure de 3-méthyl-N-butylpyridinium,
le chlorure de 4-méthyl-N-hexylpyridinium,
le méthylsulfate de 1,3-diméthylimidazolium,
le méthylsulfate de 1-méthyl-3-butylimidazolium,
l'acétate de 1-éthyl-3-méthylimidazolium,
le sulfate de 1-éthyl-3-méthylimidazolium,
le bromure de 1-butylpyridinium,
le trifluorométhanesulfonate de 1-butylpyrimidinium,
le trifluorométhanesulfonate de 1-hexylpyrimidinium,
le trifluoroacétate de 1-éthyl-3-méthylimidazolium,
le chlorure de trihexyl-tétradécyl-phosphonium,
le chlorure de tributyl-tétradécyl-phosphonium,
le trifluoroacétate de 1-éthyl-3-méthylimidazolium.
le chlorure de 1-hexyl-2,3-diméthylimidazolium,
le chlorure de 1-éthyl-2,3-diméthylimidazolium,
le dicyanamide de 1-éthyl-3-méthylimidazolium,
l'hydroxyde de tétrabutylammonium,
le salicylate de choline,
le méthylsulfate de tributylméthylammonium,
l'acétate de cholinium,
l'acétate de tétraéthylammonium tétrahydraté,
le dibutylphosphate de triéthylméthylammonium,
le L-(+)lactate de 1-éthyl-3-méthylimidazolium,
et leurs hydrates.

8. Procédé selon l'une quelconque des revendications précédentes dans lequel B⁻Y⁺ est choisi parmi les composés suivants :
Hexafluorophosphate d'ammonium, ou de métaux alcalins
Tétrafluoroborate d'ammonium, ou de métaux alcalins
Bistriflimidure d'ammonium ou de métaux alcalins
Octylsulfate d'ammonium ou de métaux alcalins.

9. Procédé selon l'une quelconque des revendications précédentes dans lequel A⁺B⁻ est choisi parmi l'hexafluorophosphate de 3-méthyl octylimidazolium, l'hexafluorophosphate de 3-méthyl butylimidazolinium, l'hexafluorophosphate de 1-éthyl-3-méthylimidazolium, l'hexafluorophosphate de 1-hexyl-3-méthylimidazolium, l'hexafluorophosphate de 1-décyl-3-méthylimidazolium, l'hexafluorophosphate de 1-dodécyl-3-méthylimidazolium, l'hexafluorophosphate de 1,3-diméthylimidazolium, l'hexafluorophosphate de de 4-méthyl-N-butyl-pyridinium, l'hexafluorophosphate de 3-méthyl-N-butylpyridinium, l'hexafluorophosphate de 4-méthyl-N-hexylpyridinium, l'hexafluorophosphate de 1-éthyl-3-méthylimidazolium, l'hexafluorophosphate de 1-butylpyridinium, de l'hexafluorophosphate d' arylazodimethylimidazolium, de l'hexafluorophosphate d'arylazométhylpyridinium

10. Procédé selon l'une quelconque des revendications précédentes dans lequel on applique successivement i) puis ii).

11. Procédé selon l'une quelconque des revendications précédentes, comprenant une étape de rinçage et/ou une étape de traitement thermique, notamment avec un fer, à friser ou à lisser, à une température comprise entre 150°C et 250°C.

## Patentansprüche

1. Verfahren zur Behandlung von Keratinfasern, das Folgendes umfasst: i) das Aufbringen einer Zusammensetzung, die mindestens eine wasserlösliche erste ionische Flüssigkeit A⁺X⁻ mit einer Wasserlöslichkeit bei 25 °C von mehr als 5 Gew.-%, mit einem organischen Kation A⁺ umfasst, und ii) das Aufbringen einer Zusammensetzung, die ein wasserlösliches Salz B⁻Y⁺ mit einer Wasserlöslichkeit bei 25 °C von mehr als 5 Gew.-% mit einem Anion B⁻ umfasst, wobei das Anion B⁻ so beschaffen ist, dass sich durch Ionenaustausch mit der ersten ionischen Flüssigkeit A⁺X⁻ auf den Fasern eine zweite hydrophpobe ionische Flüssigkeit A⁺B⁻ mit einer Wasserlöslichkeit bei Umgebungstemperatur (25 °C) von weniger als 5 Gew.-% in situ bildet, wobei die ionischen Flüssigkeiten einen Schmelzpunkt kleiner oder gleich 150 °C aufweisen.

2. Verfahren nach Anspruch 1, wobei die erste ionische Flüssigkeit und die zweite ionische Flüssigkeit einen Schmelzpunkt kleiner oder gleich 50 °C und größer als 0 °C aufweisen.

3. Verfahren nach Anspruch 1 oder 2, wobei es sich bei dem Kation A⁺ um ein organisches Kation handelt, das aus Imidazolium-, Pyrazolium-, Pyridinium-, Pyrimidinium-, Tetra(C₁-C₆)alkyl-phosphonium-, Tetra(C₁-C₆)alkylammonium-, Guanidinium-, Cholinium-, Pyrrolidinium-, Uronium-, Thiouronium- und Isothiouronium-Kationen ausgewählt ist.

4. Verfahren nach Anspruch 1 oder 2, wobei das Kation Y⁺ aus Monokationen, wie denjenigen von Alkalimetallen, Aminen und Ammoniumverbindungen oder anderen stickstoffhaltigen Derivaten, wie Guanidiniumverbindungen, ausgewählt ist.

5. Verfahren nach Anspruch 1 oder 2, wobei die Anionen B⁻ aus schwach koordinierenden Anionen ausgewählt sind, die aus polyhalogenierten Alkyl- oder Polyarylanionen, Anionen mit einer hydrophoben Kohlenwasserstoffkette, langkettigen Alkylsulfaten mit mehr als 5 Kohlenstoffatomen, Halogenphosphat-Ionen, (Di/Tri)halogenacetat-Ionen, (Di/Tri)halogenmethansulfonat-Ionen und Halogensulfat-Ionen ausgewählt sind.

6. Verfahren nach Anspruch 1 oder 2, wobei die Anionen X⁻ aus Bromiden, Chloriden, kurzkettigen C₁-C₄-Carboxylaten, Nitraten, anionischen Oxidverbindungen, Tetrahalogenoaluminat-Ionen, Tetrahalogenonickel-Ionen, dem Perchlorat-Ion (ClO₄⁻), dem Nitrat-Ion (NO₃⁻); dem Nitrit-Ion (NO₂⁻) ; dem Sulfat-Ion (SO₄²⁻), dem Hydrogensulfat-Ion, dem Phosphat-Ion, Acetaten und Formiaten, Citrat- und Lactat-Ionen ausgewählt sind.

7. Verfahren nach einem der vorhergehenden Ansprüche, wobei A⁺X⁻ aus den folgenden Verbindungen ausgewählt ist:
1-Ethyl-3-methylimidazoliumchlorid,
1-Ethyl-3-methylimidazoliumbromid,
1-Butyl-3-methylimidazoliumchlorid,
1-Hexyl-3-methylimidazoliumchlorid,
1-Methyl-3-octylimidazoliumchlorid,
1-Decyl-3-methylimidazoliumchlorid,
1-Decyl-3-methylimidazoliumbromid,
1-Dodecyl-3-methylimidazoliumchlorid,
1-Methyl-3-tetradecylimidazoliumchlorid,
4-Methyl-N-butylpyridiniumchlorid,
3-Methyl-N-butylpyridiniumchlorid,
4-Methyl-N-hexylpyridiniumchlorid,
1,3-Dimethylimidazoliummethylsulfat,
1-Methyl-3-butylimidazoliummethylsulfat,
1-Ethyl-3-methylimidazoliumacetat,
1-Ethyl-3-methylimidazoliumsulfat,
1-Butylpyridiniumbromid,
1-Butylpyrimidiniumtrifluormethansulfonat,
1-Hexylpyrimidiniumtrifluormethansulfonat,
1-Ethyl-3-methylimidazoliumtrifluoracetat,
Trihexyltetradecylphosphoniumchlorid,
Tributyltetradecylphosphoniumchlorid,
1-Ethyl-3-methylimidazoliumtrifluoracetat,
1-Hexyl-2,3-dimethylimidazoliumchlorid,
1-Ethyl-2,3-dimethylimidazoliumchlorid,
1-Ethyl-3-methylimidazoliumdicyanamid,
Tetrabutylammoniumhydroxid,
Cholinsalicylat,
Tributylmethylammoniummethylsulfat,
Choliniumacetat,
Tetraethylammoniumacetattetrahydrat,
Triethylmethylammoniumdibutylphosphat,
1-Ethyl-3-methylimidazolium-L-(+)-lactat
und Hydraten davon.

8. Verfahren nach einem der vorhergehenden Ansprüche, wobei B⁻Y⁺ aus den folgenden Verbindungen ausgewählt ist:
Ammonium- oder Alkalimetallhexafluorophosphat,
Ammonium- oder Alkalimetalltetrafluoroborat,
Ammonium- oder Alkalimetallbistriflimid,
Ammonium- oder Alkalimetalloctylsulfat.

9. Verfahren nach einem der vorhergehenden Ansprüche, wobei A⁺B⁻ aus 3-Methyloctylimidazoliumhexafluorophosphat, 3-Methylbutylimidazoliniumhexafluorophosphat, 1-Ethyl-3-methylimidazoliumhexafluoro-phosphat, 1-Hexyl-3-methylimidazoliumhexafluoro-phosphat, 1-Decyl-3-methylimidazoliumhexafluoro-phosphat, 1-Dodecyl-3-methylimidazoliumhexafluoro-phosphat, 1,3-Dimethylimidazoliumhexafluorophosphat, 4-Methyl-N-butylpryidiniumhexafluorophosphat, 3-Methyl-N-butylpryidiniumhexafluorophosphat, 4-Methyl-N-hexylpryidiniumhexafluorophosphat, 1-Ethyl-3-methylimidazoliumhexafluoro-phosphat, 1-Butylpyridiniumhexafluorophosphat, Arylazodimethylimidazoliumhexafluorophosphat und Arylazomethylpyridiniumhexafluorophosphat ausgewählt ist.

10. Verfahren nach einem der vorhergehenden Ansprüche, wobei man (i) und dann (ii) nacheinander aufbringt.

11. Verfahren nach einem der vorhergehenden Ansprüche, umfassend einen Spülschritt oder einen Wärmebehandlungsschritt, insbesondere mit einem Lockenstab oder Glätteisen, bei einer Temperatur zwischen 150 °C und 250 °C.

## Claims

1. Method for treating keratin fibres which comprises i) applying a composition comprising at least one water-soluble first ionic liquid A⁺X⁻ having a water-solubility at 25°C of greater than 5% by weight comprising an organic cation A⁺ and ii) applying a composition comprising a water-soluble salt B⁻Y⁺ having a water-solubility at 25°C of greater than 5% by weight comprising an anion B⁻, the anion B⁻ being such that, by ion exchange with the first ionic liquid A⁺X⁻, it forms *in situ,* on the fibres, a hydrophobic second ionic liquid A⁺B⁻ having a water-solubility at ambient temperature (25°C) of less than 5% by weight; the ionic liquids having a melting point of less than or equal to 150°C.

2. Method according to Claim 1, in which the first ionic liquid and the second ionic liquid have a melting point of less than or equal to 50°C and greater than 0°C.

3. Method according to Claim 1 or 2, in which the cation A⁺ is an organic cation chosen from imidazolium, pyrazolium, pyridinium, pyrimidinium, tetra(C₁-C₆)alkylphosphonium, tetra(C₁-C₆)alkylammonium, guanidinium, cholinium, pyrrolidinium, uronium, thiouronium and isothiouronium cations.

4. Method according to Claim 1 or 2, in which the cation Y⁺ is chosen from monocations, such as those of alkali metals, amines and ammoniums or other nitrogenous derivatives, such as guanidiniums.

5. Method according to Claim 1 or 2, in which the anions B⁻ are chosen from weakly coordinating anions chosen from polyhalogenated alkyl or polyaryl anions, anions with a hydrophobic carbon-based chain, long-chain alkyl sulfates of more than 5 carbon atoms, halophosphate ions, (di/tri)haloacetate ions, (di/tri)halomethane sulfonate ions and halosulfate ions.

6. Method according to Claim 1 or 2, in which the anions X⁻ are chosen from bromides, chlorides, C₁-C₄ short-chain carboxylates, nitrates, anionic oxide compounds, tetrahaloaluminate ions, tetrahalonickel ions, the perchlorate ion (ClO₄⁻), the nitrate ion (NO₃⁻); the nitrite ion (NO₂⁻); the sulfate ion (SO₄²⁻), the hydrogensulfate ion, the phosphate ion, acetates and formates, and citrate or lactate ions.

7. Method according to any one of the preceding claims, in which A⁺X⁻ is chosen from the following compounds:
1-ethyl-3-methylimidazolium chloride,
1-ethyl-3-methylimidazolium bromide,
1-butyl-3-methylimidazolium chloride,
1-hexyl-3-methylimidazolium chloride,
1-methyl-3-octylimidazolium chloride,
1-decyl-3-methylimidazolium chloride,
1-decyl-3-methylimidazolium bromide,
1-dodecyl-3-methylimidazolium chloride,
1-methyl-3-tetradecylimidazolium chloride,
4-methyl-N-butylpyridinium chloride,
3-methyl-N-butylpyridinium chloride,
4-methyl-N-hexylpyridinium chloride,
1,3-dimethylimidazolium methyl sulfate,
1-methyl-3-butylimidazolium methyl sulfate,
1-ethyl-3-methylimidazolium acetate,
1-ethyl-3-methylimidazolium sulfate,
1-butylpyridinium bromide,
1-butylpyrimidinium trifluoromethanesulfonate,
1-hexylpyrimidinium trifluoromethanesulfonate,
1-ethyl-3-methylimidazolium trifluoroacetate,
trihexyltetradecylphosphonium chloride,
tributyltetradecylphosphonium chloride,
1-ethyl-3-methylimidazolium trifluoroacetate,
1-hexyl-2,3-dimethylimidazolium chloride,
1-ethyl-2,3-dimethylimidazolium chloride,
1-ethyl-3-methylimidazolium dicyanamide,
tetrabutylammonium hydroxide,
choline salicylate,
tributylmethylammonium methyl sulfate,
cholinium acetate,
tetraethylammonium acetate tetrahydrate,
triethylmethylammonium dibutylphosphate,
1-ethyl-3-methylimidazolium L-(+)-lactate,
and hydrates thereof.

8. Method according to any one of the preceding claims, in which B⁻Y⁺ is chosen from the following compounds:
ammonium or alkali metal hexafluorophosphate,
ammonium or alkali metal tetrafluoroborate,
ammonium or alkali metal bistriflimide,
ammonium or alkali metal octyl sulfate.

9. Method according to any one of the preceding claims, in which A⁺B⁻ is chosen from 3-methyloctylimidazolium hexafluorophosphate, 3-methylbutylimidazolinium hexafluorophosphate, 1-ethyl-3-methylimidazolium hexafluorophosphate, 1-hexyl-3-methylimidazolium hexafluorophosphate, 1-decyl-3-methylimidazolium hexafluorophosphate, 1-dodecyl-3-methylimidazolium hexafluorophosphate, 1,3-dimethylimidazolium hexafluorophosphate, 4-methyl-N-butylpyridinium hexafluorophosphate, 3-methyl-N-butylpyridinium hexafluorophosphate, 4-methyl-N-hexylpyridinium hexafluorophosphate, 1-ethyl-3-methylimidazolium hexafluorophosphate, 1-butylpyridinium hexafluorophosphate, arylazodimethylimidazolium hexafluorophosphate, and arylazomethylpyridinium hexafluorophosphate.

10. Method according to any one of the preceding claims, in which i) then ii) are successively applied.

11. Method according to any one of the preceding claims, comprising a rinsing step and/or a heat treatment step, in particular with a curling iron or a straightening iron, at a temperature of between 150°C and 250°C.
